(19) Europäisches Patentamt
European Patent Office
Office européen des brevets

(11) EP 1 155 676 A2

(12) **DEMANDE DE BREVET EUROPEEN**

(43) Date de publication:
**21.11.2001 Bulletin 2001/47**

(51) Int Cl.[7]: **A61K 7/035**

(21) Numéro de dépôt: **01401249.6**

(22) Date de dépôt: **15.05.2001**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **19.05.2000 FR 0006448**

(71) Demandeur: **L'OREAL**
**75008 Paris (FR)**

(72) Inventeurs:
- **Hadasch, Anke**
**75005 Paris (FR)**
- **Lemann, Patricia**
**94000 Creteil (FR)**
- **Simonnet, Jean-Tierry**
**75011 Paris (FR)**

(74) Mandataire: **Doressamy, Clarisse**
**L'Oreal,**
**D.P.I.,**
**6, rue Bertrand Sincholle**
**92585 Clichy Cedex (FR)**

(54) **Composition de maquillage et/ou de soin sous forme de poudre comprenant un liant particulier**

(57) La présente invention est relative à une composition de maquillage et/ou de soin sous forme de poudre comprenant une phase pulvérulente et un liant, caractérisée par le fait que le liant est une composition à phase continue aqueuse.

Elle porte également sur les applications cosmétiques, en maquillage et/ou en soin, d'une telle composition.

EP 1 155 676 A2

## Description

La présente invention a pour objet une composition de maquillage et/ou de soin sous forme de poudre, par exemple libre, compacte, pressée ou encore coulée, comprenant un liant particulier.

Les poudres de maquillage comprennent généralement, d'une part, une phase pulvérulente comportant notamment des pigments et des charges et d'autre part, une phase grasse au titre de liant comprenant des corps gras, destinée à conférer au produit fini une certaine densité, à donner une douceur et une propriété émolliente au produit de maquillage et à favoriser son adhérence sur la peau.

L'élaboration des agents liants dans les poudres, en particulier dans les poudres compactes, soulève de nombreuses difficultés car la composition finale doit être suffisamment homogène et compacte pour présenter une bonne aptitude au prélèvement et pour éviter par ailleurs une fragmentation provoquée notamment par des chocs.

Il est possible d'utiliser comme liant un mélange d'huiles minérales et végétales associées à des esters. Cependant, les produits contenant ces esters peuvent mener à des produits manquant de douceur, voire à une mauvaise dispersion de la phase pigmentaire.

Il est également possible d'utiliser des huiles de silicones qui peuvent apporter du glissant, de la douceur, de la facilité d'étalement mais les propriétés de tenue du maquillage et de résistance au choc sont médiocres.

Enfin, certaines huiles perfluorées, en particulier les perfluoropolyéthers, sont bien connues comme apportant de la douceur aux compositions cosmétiques, mais le formulateur se trouve alors confronté à des problèmes d'insolubilité de ces huiles dans les huiles hydrocarbonées usuelles ou les huiles de silicones, cette insolubilité entraînant des problèmes d'instabilité de la composition de maquillage et/ou de soin. Par ailleurs, les compositions comprenant des huiles perfluorées présentent de médiocres propriétés de résistance au choc. Certaines de ces huiles peuvent également présenter l'inconvénient d'une mauvaise dispersion de la phase pigmentaire: la teinte apportée par le pigment est relativement peu intense, pouvant provoquer un « effet blanc », ce qui n'est pas souhaité, esthétiquement parlant.

Les poudres de maquillage sont donc des produits constitués généralement d'un très fort taux de composés pulvérulents secs et d'huile. Ces produits peuvent ainsi donner des sensations de tiraillement ou un effet desséchant lorsqu'ils sont appliqués sur la peau. Toutefois, du fait du fort taux de composés pulvérulents, il est très difficile d'introduire dans de tels produits des quantités significatives d'eau et d'obtenir un produit poudreux homogène. En effet, l'eau ne lie pas les particules, elle s'agglomère avec les composés pulvérulents pour former une sorte de pâte hétérogène et instable ou gâchage dont l'utilisation en tant que produit de maquillage est impossible : un prélèvement dosé de produit ne peut pas se faire. Par ailleurs, on n'obtient plus un produit poudré destiné à donner au teint un aspect velouté mais une pâte collante donnant un effet plâtre.

Un autre inconvénient de la difficulté d'introduire de l'eau dans les poudres de maquillage est l'impossibilité d'incorporer dans les poudres des agents actifs, en particulier hydrophiles.

Or, de plus en plus, les utilisatrices souhaitent des produits de maquillage qui non seulement les embellissent mais qui apportent également de la fraîcheur ou du soin à leur peau.

Ainsi, il serait particulièrement utile de pouvoir introduire dans les poudres de maquillage une phase aqueuse.

La Demanderesse a trouvé de manière inattendue que l'utilisation comme liant de poudre d'une composition à phase continue aqueuse permettait l'obtention d'une poudre qui non seulement apporte de la fraîcheur et de l'hydratation mais qui de plus présente d'excellentes propriétés cosmétiques, en soin et/ou maquillage.

L'invention a donc pour objet une composition de maquillage et/ou de soin sous forme de poudre comprenant une phase pulvérulente et un liant, caractérisée par le fait que le liant est une composition à phase continue aqueuse.

Les compositions selon l'invention présentent l'avantage de pouvoir comprendre des quantités significatives d'eau ou d'actifs hydrophiles tout en restant des poudres aisément délitables et très confortables à l'application. Malgré la présence d'eau ou d'actifs hydrophiles, on n'observe pas de formation de pâte hétérogène à effet plâtre. On obtient une poudre cosmétique présentant une bonne cohésion et que l'on peut utiliser facilement pour se maquiller.

Les compositions ainsi obtenues provoquent une agréable sensation de fraîcheur à l'application. Elles hydratent la peau et ne procurent pas d'effet de tiraillement ou de desséchement. Elles présentent également une excellente dispersion des pigments. La composition obtenue est très homogène et elle le reste même après application sur la peau, et ce pendant plusieurs heures.

Les compositions selon l'invention présentent également d'excellentes propriétés cosmétiques : elles adhèrent suffisamment à la peau mais pas trop, elles sont très douces, elles s'appliquent facilement.

Les compositions sous forme de poudre ainsi obtenues présentent une excellente tenue. Elles ne transfèrent pas et ne migrent pas non plus dans les plis de la peau.

La présente invention a encore pour objet un procédé cosmétique de maquillage ou de soin des matières kératiniques des êtres humains, en particulier de la peau et du corps, comprenant l'application sur ces matières de la composition telle que définie ci-dessus.

Par matières kératiniques, on entend selon la présente invention, la peau, les ongles, les cheveux, les cils et

sourcils, les muqueuses (intérieurs des paupières inférieures) et les semi-muqueuses (lèvres), et toute autre zone cutanée du corps et du visage.

La présente invention a encore pour objet l'utilisation d'au moins une composition à phase continue aqueuse, dans une composition de maquillage et/ou de soin sous forme de poudre, dans le but d'améliorer l'hydratation sur les matières kératiniques conférée par ladite composition.

La présente invention a également pour objet l'utilisation d'au moins une composition à phase continue aqueuse, dans une composition de maquillage et/ou de soin sous forme de poudre, dans le but d'améliorer le développement de la couleur au sein de ladite composition.

Ainsi, il est possible, grâce à la présente invention, de réaliser des compositions comprenant moins de pigments que les compositions de l'art antérieur tout en obtenant la même intensité en couleur. De même, en utilisant le même taux de pigments qu'une composition de l'art antérieur, l'utilisation du liant selon l'invention permet d'obtenir une composition dont la couleur est beaucoup plus vivace, plus intense, plus développée.

Les compositions selon l'invention trouvent notamment une application particulièrement intéressante dans le domaine du maquillage et/ou du soin de la peau et des muqueuses. Ainsi, l'invention trouve une application toute particulière dans le domaine des produits de maquillage du visage et de la peau, tels que les fards à paupières, les fards à joues, les poudres du visage et du corps, les anticernes, les produits de maquillage du corps.

D'autres caractéristiques, aspects et avantages de la présente invention apparaîtront à la lecture de la description détaillée qui va suivre.

Les compositions selon l'invention comprennent un liant qui est une composition à phase continue aqueuse, c'est-à-dire une composition comprenant une phase continue aqueuse, et qui peut représenter jusqu'à 30% en poids, de préférence de 0,1 à 23% en poids, et préférentiellement encore de 3 à 20% en poids, par rapport au poids total de la composition.

La phase aqueuse du liant selon l'invention peut comprendre de l'eau ou une eau florale telle que l'eau de bleuet.

La phase aqueuse peut également comprendre de 0 % à 15 % en poids, par rapport au poids total de la phase aqueuse, d'un monoalcool inférieur en $C_2$-$C_6$ et/ou d'un polyol tel que le glycérol, le butylèneglycol, l'isoprèneglycol, le propylèneglycol.

La phase aqueuse peut également comprendre un ou plusieurs gélifiants. Parmi ces gélifiants, on peut citer les polymères hydrophiles comme les copolymères acide acrylique/acrylate d'éthyle et les polymères carboxyvinyliques. Des exemples de tels polymères ou copolymères sont notamment les "carbomer" (CTFA) vendus par la société GOODRICH sous la dénomination Carbopol ou le polyglycérylméthacrylate vendu par la société GUARDIAN sous la dénomination Lubragel ou encore le polyglycérylacrylate vendu sous la dénomination Hispagel par la société HISPANO CHIMICA ou enfin le mélange polyacrylamide/$C_{13}$-$C_{14}$ Isoparaffin/Laureth7 vendu par la société SEPPIC sous la dénomination Sepigel.

Comme autres gélifiants utilisables dans l'invention, on peut citer : les dérivés de polysaccharides, tels que les dérivés cellulosiques comme la carboxyméthylcellulose, l'hydroxyméthylcellulose, l'hydroxypropylcellulose, la cellulose microcristalline ; les polymères de xanthane, de gellane, de rhamsan, les alginates, la maltodextrine, l'amidon et ses dérivés, l'acide hyaluronique et ses sels, la gomme de karoya, la farine de caroube, les dérivés de guar notamment l'hydropropylguar.

On peut encore utiliser comme polymère hydrophile les polyéthylèneglycols (PEG) et leurs dérivés, les polyvinylpyrrolidones et leurs dérivés.

Dans une forme préférée de réalisation de l'invention, le liant est une émulsion H/E stabilisée par un ou plusieurs systèmes organisés. Par systèmes organisés, on entend au sens de la présente invention des micelles inverses ou des structures « cristal liquide lyotrope » qui sont formées à température ambiante par le mélange de plusieurs tensio-actifs ou le mélange de tensio-actifs et de solvants polaires ou le mélange de plusieurs solvants polaires, les solvants polaires étant par exemple choisis parmi l'eau, le glycérol, le panthénol, le propylène glycol, le butylène glycol et/ou leurs mélanges. L'état cristallin liquide est un état intermédiaire entre l'état solide et l'état liquide. Il est souvent appelé état mésomorphe. Les systèmes organisés de la présente invention sont thermodynamiquement stables. Ils sont de préférence choisis parmi les cristaux liquides lyotropes. L'état cristal liquide lyotrope peut être caractérisé par l'homme du métier par des moyens connus tels que la microscopie électronique en transmission (après coloration négative ou cryofracture) ou la biréfringence sous lumière polarisée et diffraction de rayons X. Les cristaux liquides lyotropes peuvent comprendre les cristaux liquides hexagonaux inverses, également connus sous le nom de phase hexagonale II ou phase F, les cristaux liquides cubiques, également connus sous le nom de phase $I_2$, les cristaux liquides lamellaires, également connus sous le nom de phase $L_a$ et phase D.

De préférence, les cristaux liquides lyotropes utilisés dans la présente invention sont choisis parmi les cristaux liquides cubiques, les cristaux liquides lamellaires et leurs mélanges.

Dans une première forme de réalisation de l'invention, le liant est une émulsion H/E stabilisée par un système organisé comprenant des, de préférence constitué de, cristaux liquides cubiques formant un gel. Ainsi, le liant est une

émulsion stabilisée par une dispersion de particules de gel cubique.

Le terme de gel cubique utilisé selon la présente invention désigne des gels transparents, isotropes en lumière polarisée, se présentant sous forme de phase cristal liquide cubique. Les phases cubiques sont organisées d'une manière bi-polaire en domaines hydrophile et lipophile distincts, en contact étroit et formant un réseau tridimensionnel thermodynamiquement stable. Une telle organisation a été notamment décrite dans « La Recherche », Vol.23, pp. 306-315, Mars 1992 et dans « Lipid Technology », Vol.2, n° 2, pp.42-45, Avril 1990. Selon l'arrangement des domaines hydrophile et lipophile, la phase cubique est dite de type normal ou inverse. Le terme de gel cubique utilisé selon la présente invention regroupe bien entendu les gels présentant les différents types de phases cubiques.

De tels gels cubiques sont par exemple décrits dans EP 0 686 386, EP 0 711 540 et EP 0 968 704.

Dans cette forme préférée de réalisation de l'invention, le liant est sous la forme d'une dispersion, à phase continue aqueuse , de particules de gel cubique à base de phytantriol comprenant :

(a) de 0,1 à 15 % en poids de 3,7,11,15-tétraméthyl 1,2,3-hexadecanetriol ou phytantriol par rapport au poids total du liant, et

(b) de 0,1 à 3 % en poids d'un agent dispersant et stabilisant par rapport au poids total du liant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

Selon un mode de réalisation préféré, la proportion en phytantriol est comprise entre 0,5 et 10 % en poids par rapport au poids total du liant.

De préférence, le rapport pondéral entre le phytantriol et ledit agent dispersant et stabilisant tel que défini précédemment est compris entre 1 et 200, et de façon particulièrement préférée, entre 2 et 50.

Le phytantriol est un composé connu qui est notamment commercialisé sous la dénomination de « Phytantriol-63926 »® par la Société ROCHE.

Dans une deuxième forme préférée de l'invention, le liant est une composition sous forme d'une dispersion comprenant :

($\alpha$) de 60 à 98 % en poids d'une phase aqueuse, et

($\beta$) de 2 à 40 % en poids d'une phase huileuse,

ladite phase huileuse étant dispersée dans ladite phase aqueuse et stabilisée à l'aide de particules de gel cubique, lesdites particules étant formées d'au moins, de préférence essentiellement formées par, :

(i) 0,1 à 15 % en poids par rapport au poids total de la composition, d'au moins un composé choisi parmi le 3,7,11,15-tétraméthyl-1,2,3-hexadecanetriol ou phytantriol, les dérivés N-2-alcoxycarbonyles de N-méthylglucamine et les monoglycérides d'acide gras insaturé, et

(ii) 0,05 à 3 % en poids par rapport au poids total de la composition d'un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

De préférence, la proportion pondérale relative en composé (i) par rapport au poids de la phase huileuse est comprise entre 0,02/1 et 1/1, et de préférence comprise entre 0,05/1 et 0,5/1.

Selon un mode de réalisation particulier des compositions selon l'invention, la proportion pondérale relative en composé (i) par rapport au poids dudit agent dispersant et stabilisant est comprise entre 2 et 200, et de préférence inférieure ou égale à 50.

Parmi les dérivés N-2-alcoxycarbonyles de N-méthylglucamine, on peut notamment citer ceux répondant à la formule (I) suivante :

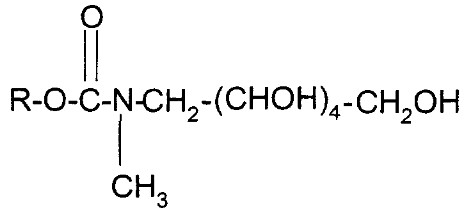

(I)

dans laquelle :
R représente un radical alkyle ramifié en $C_6$-$C_{18}$.

Parmi ceux-ci, on peut notamment mentionner la N-2-hexyldécyloxycarbonyl-N-méthyl-glucamine, la N-2-éthylhexyloxycarbonyl-N-méthyl-glucamine et la N-2-butyloctyloxycarbonyl-N-méthyl-glucamine.

La méthode de préparation des composés de formule (I) est décrite dans EP 0 711 540.

Selon un mode de réalisation particulier des liants selon l'invention, les particules de gel cubique contiennent comme composé (i), un mélange de phytantriol en une proportion de 1 à 40 % en poids par rapport au poids du mélange, et d'au moins un dérivé N-2-alcoxycarbonyle de N-méthyl-glucamine de formule (I) en une proportion de 60 à 99 % en poids par rapport au poids du mélange.

Selon une forme préférée de ce mode de réalisation, la proportion en phytantriol est de 10 à 30 % en poids par rapport au poids du mélange et celle du dérivé N-2-alcoxycarbonyle de N-méthyl-glucamine de 70 à 90 % en poids par rapport au poids du mélange.

Les monoglycérides d'acide gras insaturé sont de préférence ceux ayant une chaîne grasse insaturée en $C_{16}$-$C_{22}$.

Parmi ceux-ci, on peut notamment citer le monooléate de glycéryle ou monooléine et le monolinoléate de glycéryle ou monolinoléine.

On peut bien entendu utiliser dans les compositions selon l'invention, un mélange de monoglycérides tels que définis précédemment ainsi qu'un mélange de monoglycérides d'acide gras insaturé et de monoglycérides d'acide gras saturé, la proportion en monoglycérides d'acide gras saturé étant cependant de préférence inférieure à celle de monoglycérides d'acide gras insaturé.

Selon un autre mode de réalisation des liants selon l'invention, les particules de gel cubique contiennent comme composé (i), un mélange de phytantriol en une proportion de 1 à 50 % en poids par rapport au poids total du mélange et d'au moins un monoglycéride d'acide gras insaturé en une proportion de 50 à 99 % en poids par rapport au poids du mélange.

Selon une forme préférée de ce mode de réalisation, la proportion en phytantriol est de 10 à 30 % en poids par rapport au poids du mélange et celle en monoglycéride d'acide gras insaturé de 70 à 90 % en poids par rapport au poids du mélange.

L'agent dispersant et stabilisant tel que défini précédemment est de préférence choisi parmi :

- les alkyl ou alcényl éthers ou esters de polyol,
- les amino-acides N-acylés et leurs dérivés et les peptides N-acylés par un radical alkyle ou alcényle, et leurs sels,
- les alkyl ou alcényl éthers ou esters sulfates, leurs dérivés et leurs sels,
- les alkyl ou alcényl éthers ou esters gras polyoxyéthylénés,
- les acides alkyl ou alcényl carboxyliques polyoxyéthylénés et leurs sels,
- les N-alkyl ou alcényl bétaïnes,
- les alkyl ou alcényl triméthylammonium et leurs sels, et
- leurs mélanges.

Dans les composés ci-dessus énumérés, les radicaux alkyle et alcényle ont de 8 à 22 atomes de carbone et peuvent se présenter sous forme de mélanges.

Dans cette forme de réalisation de l'invention, le liant a un pH généralement compris entre 5 et 8 et de préférence compris entre 6 et 7.

Les liants selon l'invention ainsi réalisés sont stables et peuvent être conservés pendant 2 mois à une température comprise entre 4 et 45 °C, sans présenter aucune variation d'aspect macroscopique ni microscopique, ni de couleur, ni d'odeur.

De préférence, les particules de gel cubique comprennent en outre de 0,0005 % à 5 % en poids et de préférence de 0,001 % à 2 % en poids d'un lipide amphiphile ionique non hydrosoluble.

Parmi ceux-ci, on peut notamment citer les phopholipides naturels, modifiés ou de synthèse, les esters phosphoriques d'alcool gras, les dérivés N-acylés de l'acide glutamique, le cétylsulfate de sodium, le cocoyl monoglycéride sulfate de sodium et les dérivés d'ammonium quaternaire et/ou leurs mélanges.

Les liants sous forme de dispersion de particules de gel cubique telles que définies précédemment sont obtenus par fragmentation, à l'aide d'un homogénéiseur, d'un gel cubique à base de phytantriol, d'eau, d'au moins un agent tensioactif hydrosoluble à chaîne grasse tel que défini précédemment et éventuellement de lipides amphiphiles ioniques non hydrosolubles tels que définis précédemment et/ou de principes actifs hydrophiles et lipophiles.

Les particules de gel cubique peuvent être obtenues par divers moyens mécaniques appropriés tels que par exemple par un homogénéiseur de type rotor-stator à fort gradient de cisaillement comme le « Virtis », ou par un homogénéiseur haute pression fonctionnant entre 200 et 1.800 bars environ (20 à 180 Mpa).

La taille moyenne des particules de la dispersion, telle que définie précédemment, est généralement d'environ

0,05 à 1 µm, et de préférence inférieure ou égale à 0,5 µm. La granulométrie de la dispersion peut en outre être modulée par la nature et la concentration de l'agent tensioactif hydrosoluble à chaîne grasse utilisé.

A ce stade de la préparation, il est possible d'incorporer dans les particules de gel cubique des dispersions telles que définies précédemment, divers types de composés actifs. En particulier lesdites particules peuvent contenir un principe actif hydrophile ou un principe actif lipophile.

Bien entendu, grâce à la structure particulière des particules de gel cubique, il est possible d'incorporer dans celles-ci à la fois des principes actifs hydrophiles et des principes actifs lipophiles même si ceux-ci présentent une certaine incompatibilité.

Les liants sous forme de dispersion selon l'invention peuvent donc comprendre soit des particules contenant des principes actifs hydrophiles, soit des particules contenant des principes actifs liphophiles, soit des particules contenant à la fois des principes actifs hydrophiles et lipophiles tels que par exemple des filtres UV hydrophiles et lipophiles, ou un mélange de ces différentes particules.

Après la formation des particules de gel cubique, l'agent dispersant et stabilisant se trouve, généralement, à l'extérieur desdites particules.

La phase huileuse, contenant éventuellement certains additifs et/ou principes actifs lipophiles, est ensuite ajoutée à la dispersion obtenue et on soumet le mélange à une agitation mécanique qui peut par exemple être réalisée à l'aide d'un homogénéiseur de type « Virtis® ».

La phase huileuse se trouve ainsi être dispersée dans la phase aqueuse et se présente généralement sous forme de gouttelettes de taille moyenne comprise entre 0,1 micron et 10 microns.

Dans une deuxième forme de réalisation de l'invention, le liant est une émulsion H/E stabilisée par un système organisé comprenant des, de préférence constitué de, cristaux liquides lyotropes lamellaires. Le liant est ainsi une dispersion de vésicules pourvus d'un enrobage cristal liquide lamellaire. Selon que ces vésicules renferment un coeur huileux ou un coeur aqueux, on les appelle communément oléosomes ou au contraire liposomes et/ou niosomes.

Ainsi, comme dispersions de vésicules à coeur huileux utilisables selon la présente invention, on peut citer les émulsions de type huile-dans-eau formée de globules huileux pourvus chacun d'un enrobage cristal liquide lamellaire et dispersés dans une phase aqueuse, chaque globule huileux étant unitairement enrobé d'une couche monolamellaire ou oligolamellaire obtenue à partir d'au moins un agent tensio-actif lipophile, d'au moins un agent tensio-actif hydrophile et d'un composé choisi parmi i°) les acides gras et ii°) les lipides amphiphiles ioniques conférant à l'émulsion un pH allant de 5,5 à 7,5.

Le coeur huileux de ces vésicules peut être une huile, un mélange d'huiles, un actif lipophile, ou encore un actif dissous dans une huile. Les huiles pouvant être utilisées sont les huiles servant classiquement de support dans les compositions cosmétiques comme par exemple les triglycérides d'acides gras à chaînes courtes, les huiles de silicone, etc.

On entend par couche oligolamellaire une couche comprenant de 2 à 5 lamelles lipidiques. La taille moyenne des globules huileux revêtus est généralement inférieure à 500 nanomètres et, de préférence, inférieure à 200 nanomètres.

Selon un mode de réalisation préférentiel de l'invention l'agent tensioactif lipophile et l'agent tensioactif hydrophile comportent chacun au moins une chaine grasse saturée ayant plus de 12 atomes de carbone environ. Plus préférentiellement encore, cette chaine grasse contient de 16 à 22 atomes de carbone.

Selon un autre mode de réalisation préférentiel de l'invention, l'agent tensioactif lipophile présente un HLB compris entre environ 2 et environ 5. Comme cela est bien connu, on entend par HLB (Hydrophilic-Lipophilic Balance) l'équilibre entre la dimension et la force du groupe hydrophile et la dimension et la force de groupe lipophile de l'agent tensioactif.

Des exemples de tels agents tensioactifs lipophiles sont le distéarate de sucrose, le distéarate de diglycéryle, le tristéarate de tétraglycéryle, le décastéarate de décaglycéryle, le monostéarate de diglycérol, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthylèné 2 OE (comportant 2 motifs oxyéthylène), le mono et dibéhénate de glycéryle, le tétrastéarate de pentaérythrytol.

L'agent tensioactif hydrophile présente de préférence un HLB compris entre environ 8 et environ 12.

On peut citer comme exemples de tels tensioactifs hydrophiles les composés suivants : le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE et le distéarate polyoxyéthylèné 12 OE, le distéarate de méthylglucose polyoxyéthyléné 20 OE.

Les acide gras mis en oeuvre dans le cadre de la présente invention sont de préférence des acides gras saturés ayant de 16 à 22 atomes de carbone. On préfère ainsi utiliser l'acide palmitique, l'acide stéarique, l'acide arachidique et l'acide béhénique.

Le lipide amphiphile ionique mis en oeuvre dans le cadre de la présente invention est de préférence choisi parmi les lipides anioniques neutralisés, les lipides amphotères, les dérivés alkylsulfoniques et leurs mélanges.

Les lipides anioniques neutralisés sont choisis en particulier parmi :

- les sels alcalins du dicétylphosphate, et en particulier les sels de sodium et de potassium ;
- les sels alcalins du dimyristylphosphate, et en particulier les sels de sodium et de potassium ;
- les sels alcalins du cholestérol sulfate, et en particulier le sel de sodium ;
- les sels alcalins du cholestérol phosphate, et en particulier le sel de sodium ;
- les sels monosodique et disodique des acides acylglutamiques, et en particulier les sels monosodique et disodique de l'acide N-stéaroyl glutamique,
- le sel de sodium de l'acide phosphatidique.

Les lipides amphotères sont choisis en particulier parmi les phospholipides et notamment la phosphatidyléthanolamine de soja pure.

Les dérivés alkylsulfoniques sont avantageusement les composés de formule :

$$\begin{array}{l} R\text{-}CH\text{-}CO\text{-}O\text{-}(CH_2CH_2O)_2\text{-}CH_3 \\ \quad\ | \\ \quad SO_3M \end{array}$$

dans laquelle R représente les radicaux $C_{16}H_{33}$ et $C_{18}H_{37}$ pris en mélange ou séparément et M est un métal alcalin, de préférence le sodium.

L'enrobage selon l'invention des globules huileux demande de préférence l'utilisation d'une quantité totale d'agent tensioactif hydrophile, d'agent tensioactif lipophile et d'acide gras oude lipide amphiphile ionique comprise entre environ 2% et environ 6% en poids par rapport au poids total du liant. Encore plus préférentiellement, cette quantité est comprise entre 3% et 4%. Les quantités relatives de 1°) tensioactif lipophile, 2°) tensioactif hydrophile et 3°) d'acide gras ou respectivement de lipide amphiphile ionique varient de préférence dans les fourchettes respectives suivantes : 35-55% / 25-40% / 15-35% en poids par rapport à leur poids total.

La phase grasse, c'est à dire les gouttelettes huileuses enrobées, représente de préférence 5 à 50% en poids par rapport au poids total du liant. Encore plus préférentiellement ce pourcentage est compris entre 10 et 40.

Le rapport pondéral des globules huileux aux éléments constitutifs de l'enrobage est de préférence de 2 à 13 ; encore plus préférentiellement ce rapport est égal à 7 environ.

Lorsqu'on utilise un acide gras dans la réalisation de ces vésicules, il est préférable que la phase aqueuse du liant comprenne un agent basique destiné à la neutralisation de l'acide gras présent dans la phase huileuse. Cet agent basique doit donc être présent en une quantité au moins égale à celle nécessaire à la neutralisation de la totalité de l'acide gras. On peut utiliser comme agent basique, par exemple, la soude, la triéthanolamine, la lysine ou bien encore l'arginine.

De telles dispersions ainsi que leur procédé de préparation sont décrits dans les documents EP-A-0 641 557 et EP-A-0 705 593 .

Les vésicules à coeur aqueux pourvus d'un enrobage cristal liquide lamellaire utilisables selon la présente invention peuvent par exemple être obtenus à partir de lipides amphiphiles ayant pour formule générale :

$$X\text{-}Y$$

dans laquelle X représente un groupe hydrophile et Y représente un groupe lipophile. Les lipides amphiphiles peuvent être des lipides ioniques, pour lesquels le groupe X est ionique, ou des lipides non ioniques pour lesquels le groupe X est non ionique. On peut utiliser pour la fabrication des vésicules des mélanges de lipides amphiphiles ioniques, des mélanges de lipides amphiphiles non ioniques et des mélanges de ces deux types de lipides.

Des dispersions de vésicules utilisables pour la présente invention sont par exemples décrites dans le document FR-A-2 315 991, FR-A-2 408 387 , et FR-A-2 485 921 .

Comme dispersions de vésicules utilisables selon la présente invention, on peut également citer les dispersions aqueuses de vésicules constituées par une membrane de phase lipidique encapsulant une phase aqueuse, la phase lipidique comprenant des lipides amphiphiles non ioniques et au moins un lipide amphiphile ionique telles que :

- les lipides amphiphiles non ioniques sont constitués par un mélange d'esters d'au moins un polyol choisi dans le groupe formé par le polyéthylèneglycol comportant de 1 à 60 unités oxyde d'éthylène, le sorbitane, le sorbitane portant 2 à 60 unités oxyde d'éthylène, le glycérol portant 2 à 30 unités oxyde d'éthylène, les polyglycérols comportant 2 à 15 unités glycérol, les sucroses, les glucoses portant 2 à 30 unités oxyde d'éthylène, et d'au moins un acide gras comportant une chaîne alkyle en C5-C17, saturée ou non saturée, linéaire ou ramifiée, le nombre de chaînes alkyle par groupe polyol étant compris entre 1 et 10 ; et

- les lipides amphiphiles ioniques sont choisis dans le groupe formé par ceux conférant à la dispersion un pH compris entre 5,5 et 7,5 ;

- le rapport en poids entre la quantité de lipides amphiphiles non ioniques et celle de lipides amphiphiles ioniques dans la phase lipidique étant compris entre 50/1 et 50/25 et le rapport en poids entre la phase lipidique et la phase aqueuse de dispersion étant compris entre 1/1000 et 300/1000.

Les lipides amphiphiles ioniques associés aux lipides amphipiles non ioniques sont de préférence choisis parmi :

- les sels alcalins du dicétyl- et du dimyristylphosphate ;
- les sels alcalins du cholestérol sulfate ;
- les sels alcalins du cholestérol phosphate ;
- les lipoaminoacides et leurs sels tels que les acylglutamates mono- et disodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les phospholipides,
- les dérivés alkylsulfoniques notamment de formule :

$$\begin{array}{l} R\text{-}CH\text{-}CO\text{-}O\text{-}(CH_2\text{-}CH_2\text{-}CO)\text{-}CH_3 \\ \quad\;| \\ \quad SO_3M \end{array}$$

  dans laquelle R représente des radicaux alkyle en $C_{16}$-$C_{22}$, en particulier les radicaux $C_{16}H_{33}$ et $C_{18}H_{37}$ pris en mélange ou séparément et M est un métal alcalin ou alcalino-terreux tel que le sodium,
- leurs mélanges.

On peut, de façon connue, incorporer dans la phase lipidique constituant la membrane lipidique des vésicules, au moins un additif qui a pour fonction principale de diminuer la perméabilité des vésicules, de prévenir leur floculation et leur fusion et d'augmenter le taux d'encapsulation. Selon l'invention, on peut ajouter à la phase lipidique au moins un additif choisi, de préférence, parmi :

- les stérols, et notamment les phytostérols et le cholestérol,
- les alcools et diols en $C_{14}$ à $C_{30}$,
- les amines en $C_{14}$ à $C_{30}$ et leurs dérivés ammonium quaternaire,
- leurs mélanges.

Les vésicules de cette forme de réalisation de l'invention ont généralement un diamètre moyen compris entre 10 et 5000 nm. Lorsque la phase aqueuse comprend une dispersion de gouttelettes de liquide non miscible à l'eau, ces gouttelettes ont de façon avantageuse un diamètre moyen compris entre 100 et 10 000 nm.

Les vésicules peuvent contenir, de façon connue, un ou plusieurs composés actifs. Si les actifs sont hydrosolubles, ils sont introduits dans la phase aqueuse encapsulée des vésicules. Si les actifs sont liposolubles, on les introduits dans la phase lipidique constituant la membrane. Si les actifs sont amphiphiles, ils se répartissent entre la phse lipidique et la phase aqueuse encapsulée avec un coefficient de partage, qui varie selon la nature de l'actif amphiphile et les compositions respectives de la phase lipidique et de la phase aqueuse encapsulée.

La phase aqueuse du liant de cette forme de réalisation de l'invention peut également contenir une dispersion de gouttelettes d'une phase huileuse classique.

De telles dispersions ainsi que leur procédé de préparation sont décrits dans le document EP-A-0 582 503 .

Des dispersions utilisables selon la présente invention peuvent également être des émulsions d'une phase huileuse dans une phase aqueuse comportant des globules d'huile ayant un diamètre inférieur à 500 nanomètres, l'émulsion contenant des particules de polymère ionique, le rapport entre la quantité de particules de polymère et la

quantité de phase huileuse allant de 1/5 à 1/40 : de telles émulsions et leur procédé de préparation sont décrits dans le document EP-A-0 864 320 .

Le liant de la présente invention peut également être sous la forme de toute dispersion d'huile dans de l'eau, de toute émulsion simple H/E ou multiple E/H/E ou encore sous la forme d'une nanoémulsion.

Le liant peut également comprendre au moins un tensioactif de HLB (balance hydrophile-lipophile) supérieur ou égal à 8. De préférence, ce tensio-actif présente une réduction minimale de la tension superficielle de l'eau à la Concentration Micellaire Critique du tensio-actif : ainsi sa tension superficielle $\gamma_{sup}$, mesurée selon les méthodes classiques connues de l'homme du métier, est de préférence supérieure ou égale à 50 mN/m.

Comme tensioactif convenant particulièrement bien à la présente invention, on peut citer notamment (CTFA) : l'acide stéarique combiné à la triéthanolamine, le mélange de mono et distéarate de glycérol, le lauroyl sarcosinate de sodium, le cétéaryl-glucoside, le PEG-40 stéarate, le sorbitan tristéarate, le sorbitan stéarate, le polysorbate 60, le mélange sorbitan stéarate/sucrose cocoate, le mélange de glycéryl stéarate/PEG-100 stéarate, le PEG-400, le stéarate de glycéryle, le mélange de PEG-6/PEG-32/glycol stéarate et leurs mélanges.

La phase huileuse des liants selon l'invention peut comprendre tout composé huileux classiquement utilisé dans les émulsions.

Parmi les huiles utilisables, on peut citer l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ; les huiles d'hydrocarbures, telles que les huiles de paraffine, le squalane, la vaseline ; les esters gras, tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyldécyle, le palmitate de 2-octyl-décyle, le myristate ou le lactate de 2-octyldodécyle, le succinate de 2-diéthyl-hexyle, le malate de diisostéaryle, le triisostéarate de glycérine ou de diglycérine ; les huiles perfluorées ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, les huiles de silicone comme les polydiméthylsiloxanes, les alkylméthylpolysiloxanes et en particulier les polyalkyl($C_1$-$C_{20}$)siloxanes comme les huiles de silicone phénylées ou la cétyldiméthicone (dénomination CTFA), les cyclocopolymères du type diméthylsiloxane/méthylalkyl-siloxane, les polydiméthylsiloxane organomodifiés comme les polydiméthylsiloxane oxyéthyléné oxypropyléné, et leurs mélanges.

Le liant peut également comprendre un corps gras choisi parmi les cires et/ou les corps gras pâteux.

On peut définir les composés gras pâteux à l'aide d'au moins une des propriétés physico-chimiques suivantes :

- une viscosité de 0,1 à 40 Pa.s (1 à 400 poises), de préférence 0,5 à 25 Pa.s, mesurée à 40°C avec un viscosimètre rotatif CONTRAVES TV équipé d'un mobile MS-r3 ou MS-r4 à la fréquence de 240 tours/minute,
- un point de fusion de 25-70°C, de préférence 25-55°C.

Parmi les cires utilisables, on peut citer les cires d'abeille, les cires de lanoline et les cires d'insectes de Chine ; les cires de Carnauba, de Candelila, d'ouricurry, les cires de fibres de liège, les cires de canne à sucre, les cires du Japon, les cires de jojoba hydrogénées et les huiles hydrogénées telles que l'huile de tournesol hydrogénée, l'huile de ricin hydrogénée, l'huile de coprah hydrogénée et la lanoline hydrogénée ; les paraffines, les cires microcristallines, les cires de Montan et les ozokérites ; les cires de polyéthylène, les cires obtenues par la synthèse de Fisher-Tropsch, les copolymères cireux ainsi que leurs esters, et les cires de silicone telles que les polyalcoxy et polyalkylsiloxanes.

Le liant peut éventuellement comprendre une partie volatile comme par exemple des huiles volatiles.

On entend par huile volatile, tout composé susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.

Ces composés volatils peuvent être choisis en particulier parmi les huiles hydrocarbonées telles que les isoparaffines et notamment l'isododécane, les silicones volatiles cycliques ayant de 3 à 8 atomes de silicium et de préférence 4 à 6, comme par exemple le cyclotétra-diméthylsiloxane, le cyclopentadiméthylsiloxane ou le cyclohexadiméthylsiloxane, notamment les produits vendus sous les dénominations de "DC Fluid 244", "DC Fluid 245", "DC FLuid 344 et "DC Fluid 345" par la Société Dow Corning, les silicones volatiles linéaires ayant de 2 à 9 atomes de silicium, par exemple l'hexaméthyldisiloxane, l'hexylheptaméthyltrisiloxane et l'octylheptaméthyl-trisiloxane, et leurs mélanges.

Comme indiqué ci-dessus pour les différentes formes de réalisation de l'invention, le liant peut également comprendre des actifs. Parmi les différents actifs pouvant être incorporés, on peut notamment citer :

- les agents antioxydants ou anti-radicaux libres tels que les protéines et les enzymes, la lactoperoxydase et la lactoferrine, les peptides et leurs dérivés, les séquestrants, les flavonoïdes, la chlorophylline, l'éthoxyquine, la

guanosine, les tocophérols et leurs dérivés, le palmitate d'ascorbyle, et le β-carotène, la vitamine E et ses dérivés, la vitamine C et ses dérivés, la vitamine A et ses dérivés,

- les agents hydratants ou humectants tels que l'acide hyaluronique et son sel de sodium, le β-glycérophosphate, le glycérol, le sorbitol, le panthénol,
- les filtres UV tels que les produits commercialisés sous les dénominations de « Eusolex 232 »® par la Société MERCK, de « Parsol 1789 »® et de « Parsol MCX »® par la Société GIVAUDAN-ROURE, de « Mexoryl SX »® par la Société CHIMEX et de « UVINUL T150 »® par la Société BASF,
- les kératolytiques tels que les enzymes protéolytiques, l'acide salicylique et ses dérivés tels que l'acide n-dodécanoyl-5 salicylique, et l'acide rétinoïque et ses dérivés,
- les accélérateurs de bronzage tels que la caféine, et les dérivés de tyrosine tels que le tyrosinate de glucose et le sel disodique de la N-L-malyl tyrosine,
- les dépigmentants tels que l'acide kojique, l'acide glycolique, la vitamine C et notamment l'ascorbyle phosphate de magnésium, et l'arbutine et ses dérivés,
- les colorants naturels tels que les matières colorantes extraites de végétaux comme la chlorophylline et le β-carotène ou extraites d'animaux comme le carmin de cochenille, et le caramel,
- les auto-bronzants tels que la dihydroxyacétone, et les indoles,
- les liporégulateurs tels que le γ-orizanol, l'extrait de *Centella asiatica* contenant de la génine et de l'acide asiatique, la caféine, et la théophylline,
- les agents anti-vieillissement et anti-rides tels que les hydroxyacides comme l'acide glycolique, l'acide n-octanoyl salicylique, le rétinol et ses dérivés comme l'acétate, le palmitate et le propionate de rétinol, et les rétinoïdes,
- les agents anti-inflammatoires et cicatrisants tels que l'acide 18 β-glycyrrhétinique et ses sels comme notamment son sel d'ammonium, l'α-bisabolol, les corticoïdes, l'extrait de *Centella asiatica,* l'aloe vera
- les antibactériens et antifongiques tels que le chlorure de benzalkonium, la chlorhexidine, l'hexetidine, et l'hexamidine,
- les insectifuges tels que les diéthyl et diméthyltoluamides,
- les déodorants tels que l'hexachlorophène, et le triclosan produit commercialisé sous la dénomination de « Irgasan DP 300 »® par la Société CIBAGEIGY.
- les antipelliculaires tels que l'octopirox, et les dérivés de pyridinethione tels que ceux commercialisés sous les dénominations de « Omadine »® par la Société OLIN
- les agents anti-chute des cheveux tels que le nicotinate de méthyle ou d'hexyle, et le minoxidil,
- les colorants capillaires tels que les bases et les coupleurs d'oxydation, les colorants directs, et les colorants auto-oxydables,
- les agents réducteurs pour permanentes tels que l'acide thioglycolique, la cystéine, la cystéamine, la N-acétyl cystéine, la N-acétyl cystéamine, et le thioglycolate de glycérol,
- les agents conditionneurs pour peau et cheveux tels que les polymères cationiques et les cations.

Les compositions selon l'invention comprennent également une phase pulvérulente qui peut comprendre des pigments et/ou des nacres et/ou des charges et/ou des paillettes habituellement utilisés dans les compositions cosmétiques, et/ou leurs mélanges.
Par pigments, il faut comprendre des particules blanches ou colorées, minérales ou organiques, insolubles

dans le milieu, destinées à colorer et/ou opacifier la composition.

Les pigments peuvent être présents à une teneur allant de 0,05 à 80 % en poids, par rapport au poids total de la composition, et de préférence allant de 0,5 à 50 %. Ils peuvent être blancs ou colorés, minéraux et/ou organiques, de taille usuelle ou nanométrique. On peut citer, parmi les pigments et les nanopigments minéraux, les dioxydes de titane, de zirconium ou de cérium, ainsi que les oxydes de zinc, de fer ou de chrome, les nanotitanes, les nanozincs, le bleu ferrique. Parmi les pigments organiques, on peut citer le noir de carbone, et les laques comme les sels de calcium, de baryum, d'aluminium ou de zirconium, de colorants acides tels que les colorants halogéno-acides, azoïques ou anthraquinoniques.

Les pigments peuvent notamment être enrobés par des composés siliconés tels que des polydiméthylsiloxanes et/ou par des polymères, notamment des polyéthylènes. On peut ainsi citer les « oxydes SI » qui sont des pigments enrobés polyméthylhydrogénosiloxane vendus par la société Myioshi.

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Les charges, qui peuvent être présentes dans la composition à une teneur allant de 0,1 à 99,9 % en poids, par rapport au poids total de la composition, de préférence à une teneur allant de 70% à 99,9% et de préférence encore de 77 à 98%, peuvent être minérales ou de synthèse, lamellaires ou non lamellaires. On peut citer le talc, le mica, la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses synthétiques telles que l'Expancel (Nobel Industrie), les microéponges comme le polytrap (Dow Corning) et les microbilles de résine de silicone (Tospearls de la Société TOSHIBA, par exemple), les oxydes de zinc et de titane, les oxydes de zirconium ou de cérium, le carbonate de calcium précipité, le carbonate et l'hydrocarbonate de magnésium, l'hydroxyapatite, les microsphères de silice creuses (Silica Beads de la Société MAPRECOS), les microcapsules de verre ou de céramique ; les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Par nacres, il faut comprendre des particules irisées qui réfléchissent la lumière.

Les nacres peuvent être présentes dans la composition à une teneur allant de 0,05 à 80% en poids, de préférence à une teneur allant de 2 à 50% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Les compositions selon l'invention peuvent également comprendre des paillettes.

De préférence, la phase pulvérulente est présente dans les compositions selon l'invention à une teneur d'au moins 50% en poids, de préférence encore d'au moins 70% en poids, par rapport au poids total de la composition. De préférence encore, la phase pulvérulente est présente à une teneur allant de 77 à 99,9% en poids, par rapport au poids total de la composition.

La composition de l'invention peut comprendre, en outre, tout additif usuellement utilisé dans le domaine concerné des antioxydants, des huiles essentielles, des conservateurs, des neutralisants, des tensio-actifs E/H ou H/E, des vitamines, des actifs antirides.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter sous la forme d'une poudre, par exemple compactée, libre, pressée ou encore coulée. Dans le cas d'une poudre libre par exemple, le liant peut représenter jusqu'à 15% en poids, par rapport au poids total de la composition, de préférence de 1 à 5% en poids, par rapport au poids total de la composition. Pour une poudre compacte, la teneur en liant peut représenter de 1 à 30% en poids, de préférence de 5 à 20% en poids, par rapport au poids total de la composition.

Les compositions selon l'invention peuvent être préparées selon les méthodes connues de préparation des poudres cosmétiques.

Un procédé de préparation préféré des compositions selon la présente invention comprend les étapes suivantes :

- a°) mélange des composés pulvérulents,
- b°) broyage du mélange obtenu en a°) en particules fines,
- c°) ajout du liant et mélange jusqu'à obtention d'une composition homogène.

Le mélange de l'étape a°) peut se faire par exemple à l'aide d'un mélangeur à turbine de type « Baker-Perkins ».

Le broyage de l'étape b°) peut se faire à l'aide d'un broyeur à broches ou d'un broyeur à jet d'air. L'ajout du liant se fait de préférence goutte à goutte sous agitation dans un mélangeur dont la température est de préférence gardée constante à 35°C.

L'invention est illustrée plus en détails dans les exemples suivants.

Dans les exemples suivants, les quantités sont données en pourcentage en poids par rapport au poids total de la composition.

**EXEMPLE 1 :**

La Demanderesse a réalisé les compositions sous forme de poudres suivantes (les quantités sont données en pourcentage de poids par rapport au poids total de la composition) :

*Phase A :*

- talc X %
- oxydes de fer 2,74 %
- poudre de Nylon 10 %
- oxyde de titane 1 %
- conservateur 0,2 %

*Phase B :*

- liant (dispersion de particules de gel cubique) Y %

avec :

| | X % | Y % |
|---|---|---|
| Composition 1 | 77,06 | 9 |
| Composition 2 | 74,06 | 12 |
| Composition 3 | 71,06 | 15 |

La composition du liant (dispersion de particules de gel cubique) est la suivante (les quantités sont données en pourcentage de poids par rapport au poids total de la composition) :

- myristate d'isopropyle 1,64%
- huile de ricin 2,46%
- huile de vaseline 12,36%
- lanoline liquide 1,26%
- eau 70,95%
- imidazolinyl urée 0,3%
- glycérine 5%

- sel monosodique de N-stéaroyle-L-acide glutamique 0,03%
- phytantriol 2,97%
- vaseline 2,28%
- chlorphénésine 0,25%
- monopalmitate de sorbitane oxyéthyléné (40 OE) 0,5%

Ce liant est réalisé de la manière suivante : une dispersion aqueuse de particules de gel cubique est obtenue par mélange de 2,97 g de phytantriol et 0,03 g de sel monosodique de N-stéaroyle-L-acide glutamique et de 1,28 g d'eau, auquel on ajoute 75,17 g d'une solution aqueuse comprenant 0,5 g de monopalmitate de sorbitane oxyéthyléné et 5 g de glycérine. Le mélange est alors prédispersé puis homogénéisé à température ambiante, à l'aide d'un homogénéiseur de type « Virtis® » à 35 000 tr/min pendant 5 minutes, cette agitation étant répétée 4 fois.
A la dispersion aqueuse de particules de gel cubique obtenue, on ajoute alors les conservateurs (chlorphénésine et imidazolinyl urée) et les corps gras (myristate d'isopropyle, huile de ricin, huile de vaseline, lanoline liquide et vaseline). Après agitation à température ambiante à l'aide d'un homogénéiseur de type « Virtis ® » à 35 000 tr/min pendant 5 minutes, cette agitation étant répétée 5 fois, on obtient une dispersion stable et homogène.
Pour chaque composition 1 à 3, on prépare d'abord un mélange des composés de la phase A dans un mélangeur à turbine (type « Baker-Perkins »). Ce mélange est ensuite broyé à l'aide d'un broyeur à broches ou d'un broyeur à jet d'air. En dernier lieu, le liant (phase B) est ajouté goutte à goutte à la phase A sous agitation dans un mélangeur à turbine (type « Baker-Perkins ») dont la température est gardée constante à 35°C. Les deux phases sont mélangées jusqu'à l'obtention d'un produit homogène. On obtient des poudres présentant une bonne cohésion des composés pulvérulents.
Ces poudres procurent un effet fraîcheur à l'application sur la peau.

**EXEMPLE 2 :**

La Demanderesse a réalisé les compositions sous forme de poudres suivantes (les quantités sont données en pourcentage de poids par rapport au poids total de la composition) :

*Phase A :*

- talc X %
- oxydes de fer 2,74 %
- poudre de Nylon 10%
- oxyde de titane 1%
- conservateur 0,2 %

*Phase B :*

- liant (dispersion de vésicules à coeur huileux ayant un enrobage cristal liquide lamellaire) Y %

avec :

| | X % | Y % |
|---|---|---|
| Composition 4 | 77,06 | 9 |

(suite)

| | | |
|---|---|---|
| Composition 5 | 74,06 | 12 |
| Composition 6 | 71,06 | 15 |

La composition du liant (dispersion de vésicules à coeur huileux ayant un enrobage cristal liquide lamellaire) est la suivante (les quantités sont données en pourcentage de poids par rapport au poids total de la composition) :

*Phase 1*

- distéarate polyglycérole 2%
- monostérate de polyéthylène glycol 1,35%
- acide stéarique 1%
- vaseline 25,62%
- huile de ricin 4,305%
- myristate d'isopropyle 2,87%
- lanoline 2,205

*Phase 2*

- eau 53,9%
- tri-éthanolamine 0,25%
- glycérine 5%
- phénoxy-2-éthanol 1%
- chlorphénésine 0,25%
- alcool phényléthylique 0,25%

Préparation du liant : la phase 1 et la phase 2 sont portées, séparément à une température de 70°C. Sous agitation vive (rotor-stator), la phase 2 est introduite rapidement sur la phase 1. L'agitation et la température sont maintenues pendant 10 min. Cette émulsion est ensuite passée dans un homogénéisateur haute pression à 500b, successivement 3 fois, puis refroidie à température ambiante.
La dispersion obtenue est fluide, laiteuse et a une taille particulaire inférieure à 500 nm, de l'ordre de 220 nm.

Pour chaque composition 4 à 6, on prépare d'abord un mélange des composés de la phase A dans un mélangeur à turbine (type « Baker-Perkins »). Ce mélange est ensuite broyé à l'aide d'un broyeur à broches ou d'un broyeur à jet d'air. En dernier lieu, le liant (phase B) est ajoutée goutte à goutte à la phase A sous agitation dans un mélangeur à turbine (type « Baker-Perkins ») dont la température est gardée constante à 35°C. Les deux phases sont mélangées jusqu'à l'obtention d'un produit homogène. On obtient des poudres présentant une bonne cohésion des composés pulvérulents.

Ces poudres procurent un effet fraîcheur à l'application sur la peau.

**EXEMPLE 3 : exemple comparatif**

La Demanderesse a réalisé la composition sous forme de poudre selon l'art antérieur suivante :

Composition 7 :

*Phase A :*

- talc 73,8%
- oxyde de fer jaune 1,6%
- oxyde de fer rouge 1,7%
- oxyde de fer noir 0,5%
- poudre de nylon 10%
- nanooxyde de titane 3%
- résine méthylsilsesquioxane 5,0
- p-hydroxybenzoate de méthyle 0,3%
- carbonate de magnésium hydraté 0,2%
- parfum 0,2%

*Phase B : liant*

- polyméthylcétyldiméthylsiloxane 0,45%
- mélange de résine triméthylsiloxysilicate et de polydiméthylsiloxane 0,73%
- polydiméthylsiloxane 2,52%

La composition 7 comprend donc un liant classique à base de composé siliconés et ne comprenant pas de phase aqueuse.
La Demanderesse a également réalisé la composition 8 selon l'invention suivante :

Composition 8 :

*Phase A :*

- talc 73,8%
- oxyde de fer jaune 1,6%
- oxyde de fer rouge 1,7%
- oxyde de fer noir 0,5%

- poudre de nylon 10%
- nanooxyde de titane 3%
- résine méthylsilsesquioxane 5,0
- p-hydroxybenzoate de méthyle 0,3%
- carbonate de magnésium hydraté 0,2%
- parfum 0,2%

*Phase B :*

- liant (dispersion de vésicules ayant un enrobage cristal liquide lamellaire) 3,7%

La composition du liant de la composition 8 correspond à celle de l'exemple 2 cité ci-dessus.

Les compositions 7 et 8 ont été préparées selon les méthodes classiques. Les composés de la phase A sont mélangés dans un mélangeur à ruban. Le liant (phase B) est introduit goutte à goutte. Les phases A et B sont mélangées jusqu'à l'obtention d'un produit homogène. Ce dernier est ensuite broyé à l'aide d'un broyeur à jet d'air. La poudre obtenue est tamisée et compactée.

Les mesures des paramètres colorimétriques ont été effectuées sur les poudres compactes à l'aide d'un colorimètre Chromamètre CR-300 de Minolta (paramètres techniques : géométrie de mesure : d/0°, taille de la zone de mesure : 8 mm, source : lampe à arc xenon, illuminant D65).

Les résultats sont exprimés dans le système (L, a, b) dans lequel L représente la luminance, a représente l'axe rouge-vert (-a = vert, +a = rouge) et b représente l'axe jaune-bleu (-b =bleu, +b =jaune). Ils sont également exprimés dans le système (C, h) qui représentent les coordonnées polaires des coordonnées cartésiennes (a, b). Ainsi C est le rayon et est donc défini par la relation suivante :

$$C = \sqrt{a^2+b^2}$$

C représente ainsi la « saturation» de la couleur, c'est-à-dire sa vivacité et h représente « l'angle de teinte » de la couleur.

| **Paramètres colorimétriques** | **Composition 7** | **Composition 8** | **Δ** |
|---|---|---|---|
| L | 63,92 ± 1,63 | 65,74 ± 0,14 | 1,82 |
| a | + 12,04 ± 0,25 | + 13,81 ± 0,06 | 1,77 |
| b | + 18,33 ± 0,78 | + 21,32 ± 0,14 | 2,99 |
| c | 21,9 | 25,5 | 4,6 |
| h | 56,7 | 56,9 | 0,2 |

Les compositions 7 et 8 possèdent sensiblement le même angle de teinte h. Cela veut dire que la teinte de la couleur d'une composition à l'autre ne change pas. En revanche, pour la composition 8 dont le liant est une émulsion H/E stabilisée par des vésicules à coeur huileux comprenant une phase lamellaire et qui est donc conforme à la présente invention, on observe une augmentation significative des paramètres colorimétriques a, b et donc c, par rapport à la composition 7 dont le liant est anhydre. Cela montre que la saturation de la couleur, c'est-à-dire sa vivacité, est nettement plus élevée pour une composition comprenant un liant à phase continue aqueuse conforme à la présente invention, que pour une composition comprenant un liant anhydre tel qu'un mélange de composés siliconés.

Ainsi, il est possible, grâce à la présente invention, de réaliser des compositions comprenant moins de pigments que les compositions de l'art antérieur tout en obtenant la même intensité en couleur. De même, en utilisant le même taux de pigments qu'une composition de l'art antérieur, l'utilisation du liant selon l'invention permet d'obtenir une composition dont la couleur est beaucoup plus vivace, plus intense, plus développée.

## Revendications

1. Composition de maquillage et/ou de soin sous forme de poudre comprenant une phase pulvérulente et un liant, **caractérisée par le fait que** le liant est une composition à phase continue aqueuse.

2. Composition selon la revendication 1 telle que le liant est présent à une teneur allant jusqu'à 30%, de préférence allant de 0,1 à 23% en poids, par rapport au poids total de la composition.

3. Composition selon la revendication 1 ou 2 telle que le liant est une émulsion H/E stabilisée par un ou plusieurs systèmes organisés.

4. Composition selon la revendication 3 telle que les systèmes organisés sont choisis parmi les cristaux liquides lyotropes.

5. Composition selon la revendication 4 telle que les cristaux liquides lyotropes sont choisis parmi les cristaux liquides cubiques, les cristaux liquides lamellaires et leurs mélanges.

6. Composition selon la revendication 5 telle que le système organisé comprend des cristaux liquides cubiques formant un gel.

7. Composition selon la revendication précédente telle que le liant est sous la forme d'une dispersion, à phase continue aqueuse, de particules de gel cubique à base de phytantriol comprenant :

   (a) de 0,1 à 15 % en poids de 3,7,11,15-tétraméthyl 1,2,3-hexadecanetriol ou phytantriol par rapport au poids total du liant, et
   (b) de 0,1 à 3 % en poids d'un agent dispersant et stabilisant par rapport au poids total de la phase liante, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

8. Composition selon la revendication 6 ou 7 telle que la dispersion de particules de gel cubique est une composition sous forme d'une dispersion comprenant :

   ($\alpha$) de 60 à 98 % en poids d'une phase aqueuse, et
   ($\beta$) de 2 à 40 % en poids d'une phase huileuse,

   ladite phase huileuse étant dispersée dans ladite phase aqueuse et stabilisée à l'aide de particules de gel cubique, lesdites particules étant formées d'au moins :

   (i) 0,1 à 15 % en poids par rapport au poids total de la composition, d'au moins un composé choisi parmi le 3,7,11,15-tétraméthyl-1,2,3-hexadecanetriol ou phytantriol, les dérivés N-2-alcoxycarbonyles de N-méthylglucamine et les monoglycérides d'acide gras insaturé, et

   (ii) 0,05 à 3 % en poids par rapport au poids total de la composition d'un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

9. Composition selon la revendication 8 telle que la proportion pondérale relative en composé (i) par rapport au poids de la phase huileuse est comprise entre 0,02/1 et 1/1, et de préférence comprise entre 0,05/1 et 0,5/1.

10. Composition selon la revendication 8 telle que la proportion pondérale relative en composé (i) par rapport au poids dudit agent dispersant et stabilisant est comprise entre 2 et 200, et de préférence inférieure ou égale à 50.

11. Composition selon l'une quelconque des revendications 7 à 10 telle que l'agent dispersant et stabilisant est choisi parmi les alkyl ou alcényl éthers ou esters de polyol, les amino-acides N-acylés et leurs dérivés et les peptides N-acylés par un radical alkyle ou alcényle, et leurs sels, les alkyl ou alcényl éthers ou esters sulfates, leurs dérivés et leurs sels, les alkyl ou alcényl éthers ou esters gras polyoxyéthylénés, les acides alkyl ou alcényl carboxyliques polyoxyéthylénés et leurs sels, les N-alkyl ou alcényl bétaïnes, les alkyl ou alcényl triméthylammonium et leurs sels, et leurs mélanges.

**12.** Composition selon l'une quelconque des revendications 7 à 11 telle que les particules de gel cubique comprennent en outre de 0,0005 % à 5 % en poids et de préférence de 0,001 % à 2 % en poids d'un lipide amphiphile ionique non hydro-soluble.

**13.** Composition selon l'une quelconque des revendications 7 à 12 telle que les particules peuvent contenir un principe actif hydrophile ou un principe actif lipophile.

**14.** Composition selon la revendication 5 telle que les cristaux liquide lyotropes sont des cristaux liquides lamellaires.

**15.** Composition selon la revendication 14 telle que le liant est une dispersion de vésicules pourvus d'un enrobage cristal liquide lamellaire.

**16.** Composition selon la revendication 15 telle que les vésicules sont à coeur huileux.

**17.** Composition selon la revendication 16 telle que la dispersion de vésicules à coeur huileux pourvus d'un enrobage cristal liquide lamellaire est une émulsion de type huile-dans-eau formée de globules huileux pourvus chacun d'un enrobage cristal liquide lamellaire et dispersés dans une phase aqueuse, chaque globule huileux étant unitairement enrobé d'une couche monolamellaire ou oligolamellaire obtenue à partir d'au moins un agent tensio-actif lipophile, d'au moins un agent tensio-actif hydrophile et d'un composé choisi parmi i°) les acides gras et ii°) les lipides amphiphiles ioniques conférant à l'émulsion un pH allant de 5,5 à 7,5.

**18.** Composition selon la revendication 17 telle que l'agent tensioactif lipophile et l'agent tensioactif hydrophile comportent chacun au moins une chaine grasse saturée ayant plus de 12 atomes de carbone environ.

**19.** Composition selon la revendication 17 telle que l'acide gras est un acide gras saturé ayant de 16 à 22 atomes de carbone.

**20.** Composition selon la revendication 17 telle que le lipide amphiphile ionique est choisi parmi les lipides anioniques neutralisés, les lipides amphotères, les dérivés alkylsulfoniques, et leurs mélanges.

**21.** Composition selon la revendication 15 telle que les vésicules sont à coeur aqueux.

**22.** Composition selon la revendication précédente telle que les vésicules à coeur aqueux pourvus d'un enrobage cristal liquide lamellaire sont obtenus à partir de lipides amphiphiles ayant pour formule générale :

$$X\text{-}Y$$

dans laquelle X représente un groupe hydrophile et Y représente un groupe lipophile.

**23.** Composition selon la revendication précédente telle que les vésicules sont constitués par une membrane de phase lipidique encapsulant une phase aqueuse, la phase lipidique comprenant des lipides amphiphiles non ioniques et au moins un lipide amphiphile ionique telles que :

- les lipides amphiphiles non ioniques sont constitués par un mélange d'esters d'au moins un polyol choisi dans le groupe formé par le polyéthylèneglycol comportant de 1 à 60 unités oxyde d'éthylène, le sorbitane, le sorbitane portant 2 à 60 unités oxyde d'éthylène, le glycérol portant 2 à 30 unités oxyde d'éthylène, les polyglycérols comportant 2 à 15 unités glycérol, les sucroses, les glucoses portant 2 à 30 unités oxyde d'éthylène, et d'au moins un acide gras comportant une chaîne alkyle en C5-C17, saturée ou non saturée, linéaire ou ramifiée, le nombre de chaînes alkyle par groupe polyol étant compris entre 1 et 10 ; et

- les lipides amphiphiles ioniques sont choisis dans le groupe formé par ceux conférant à la dispersion un pH compris entre 5,5 et 7,5 ;

- le rapport en poids entre la quantité de lipides amphiphiles non ioniques et celle de lipides amphiphiles ioniques dans la phase lipidique étant compris entre 50/1 et 50/25 et le rapport en poids entre la phase lipidique et la phase aqueuse de dispersion étant compris entre 1/1000 et 300/1000.

**24.** Composition selon la revendication 23 telle que les lipides amphiphiles ioniques associés aux lipides amphipiles non ioniques sont choisis parmi :

- les sels alcalins du dicétyl- et du dimyristylphosphate ;
- les sels alcalins du cholestérol sulfate ;
- les sels alcalins du cholestérol phosphate ;
- les lipoaminoacides et leurs sels tels que les acylglutamates mono- et disodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les phospholipides,
- les dérivés alkylsulfoniques notamment de formule :

$$\begin{array}{l} R\text{-}CH\text{-}CO\text{-}O\text{-}(CH_2\text{-}CH_2\text{-}CO)\text{-}CH_3 \\ \quad\ | \\ \quad SO_3M \end{array}$$

  dans laquelle R représente des radicaux alkyle en $C_{16}$-$C_{22}$, en particulier les radicaux $C_{16}H_{33}$ et $C_{18}H_{37}$ pris en mélange ou séparément et M est un métal alcalin ou alcalino-terreux tel que le sodium,
- leurs mélanges.

**25.** Composition selon la revendication 22 à 24 telle que la phase lipidique comprend un additif choisi parmi :

- les stérols, et notamment les phytostérols et le cholestérol,
- les alcools et diols en $C_{14}$ à $C_{30}$,
- les amines en $C_{14}$ à $C_{30}$ et leurs dérivés ammonium quaternaire,
- leurs mélanges.

**26.** Composition selon la revendication 1 telle que le liant est une émulsion H/E comprenant un tensioactif de tension superficielle supérieure ou égale à 50mN/m.

**27.** Composition selon l'une quelconque des revendications précédentes telle qu'elle comprend une huile choisie parmi l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ; les huiles d'hydrocarbures, telles que les huiles de paraffine, le squalane, la vaseline ; les esters gras, tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyldécyle, le palmitate de 2-octyl-décyle, le myristate ou le lactate de 2-octyldodécyle, le succinate de 2-diéthyl-hexyle, le malate de diisostéaryle, le triisostéarate de glycérine ou de diglycérine ; les huiles perfluorées ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, les huiles de silicone comme les polydiméthylsiloxanes, les alkylméthylpolysiloxanes et en particulier les polyalkyl($C_1$-$C_{20}$)siloxanes comme les huiles de silicone phénylées ou la cétyldiméthicone (dénomination CTFA), les cyclocopolymères du type diméthylsiloxane/méthylalkyl-siloxane, les polydiméthylsiloxane organomodifiés comme les polydiméthylsiloxane oxyéthyléné oxypropyléné, et leurs mélanges.

**28.** Composition selon l'une quelconque des revendications précédentes telle qu'elle comprend un composé volatil choisi parmi les huiles hydrocarbonées telles que les isoparaffines et notamment l'isododécane, les silicones volatiles cycliques ayant de 3 à 8 atomes de silicium et de préférence 4 à 6, comme par exemple le cyclotétradiméthylsiloxane, le cyclopentadiméthylsiloxane ou le cyclohexadiméthylsiloxane, les silicones volatiles linéaires ayant de 2 à 9 atomes de silicium, par exemple l'hexaméthyldisiloxane, l'hexylheptaméthyltrisiloxane et l'octylheptaméthyl-trisiloxane, et leurs mélanges.

**29.** Composition selon l'une quelconque des revendications précédentes telle qu'elle comprend un actif choisi parmi :

- les agents antioxydants ou anti-radicaux libres tels que les protéines et les enzymes, la lactoperoxydase et

la lactoferrine, les peptides et leurs dérivés, les séquestrants, les flavonoïdes, la chlorophylline, l'éthoxyquine, la guanosine, les tocophérols et leurs dérivés, le palmitate d'ascorbyle, et le β-carotène, la vitamine E et ses dérivés, la vitamine C et ses dérivés, la vitamine A et ses dérivés,

- les agents hydratants ou humectants tels que l'acide hyaluronique et son sel de sodium, le β-glycérophosphate, le glycérol, le sorbitol, le panthénol,
- les filtres UV,
- les kératolytiques tels que les enzymes protéolytiques, l'acide salicylique et ses dérivés tels que l'acide n-dodécanoyl-5 salicylique, et l'acide rétinoïque et ses dérivés,
- les accélérateurs de bronzage tels que la caféine, et les dérivés de tyrosine tels que le tyrosinate de glucose et le sel disodique de la N-L-malyl tyrosine,
- les dépigmentants tels que l'acide kojique, l'acide glycolique, la vitamine C et notamment l'ascorbyle phosphate de magnésium, et l'arbutine et ses dérivés,
- les colorants naturels tels que les matières colorantes extraites de végétaux comme la chlorophylline et le β-carotène ou extraites d'animaux comme le carmin de cochenille, et le caramel,
- les auto-bronzants tels que la dihydroxyacétone, et les indoles,
- les liporégulateurs tels que le γ-orizanol, l'extrait de *Centella asiatica* contenant de la génine et de l'acide asiatique, la caféine, et la théophylline,
- les agents anti-vieillissement et anti-rides tels que les hydroxyacides comme l'acide glycolique, l'acide n-octanoyl salicylique, le rétinol et ses dérivés comme l'acétate, le palmitate et le propionate de rétinol, et les rétinoïdes,
- les agents anti-inflammatoires et cicatrisants tels que l'acide 18 β-glycyrrhétinique et ses sels comme notamment son sel d'ammonium, l'α-bisabolol, les corticoïdes, l'extrait de *Centella asiatica*, l'aloe vera
- les antibactériens et antifongiques tels que le chlorure de benzalkonium, la chlorhexidine, l'hexetidine, et l'hexamidine,
- les insectifuges tels que les diéthyl et diméthyltoluamides,
- les déodorants tels que l'hexachlorophène, et le triclosan,
- les antipelliculaires tels que l'octopirox, et les dérivés de pyridinethione,
- les agents anti-chute des cheveux tels que le nicotinate de méthyle ou d'hexyle, et le minoxidil,
- les colorants capillaires tels que les bases et les coupleurs d'oxydation, les colorants directs, et les colorants auto-oxydables,
- les agents réducteurs pour permanentes tels que l'acide thioglycolique, la cystéine, la cystéamine, la N-acétyl cystéine, la N-acétyl cystéamine, et le thioglycolate de glycérol,
- les agents conditionneurs pour peau et cheveux tels que les polymères cationiques et les cations.

**30.** Composition selon l'une quelconque des revendications précédentes telle que la phase pulvérulente comprend des pigments et/ou des nacres et/ou des charges et/ou des paillettes et/ou leurs mélanges.

**31.** Composition selon la revendication 30 telle que les pigments sont choisis parmi les dioxydes de titane, de zirconium ou de cérium, les oxydes de zinc, de fer ou de chrome, les nanotitanes, les nanozincs, le bleu ferrique, le noir de carbone, les laques comme les sels de calcium, de baryum, d'aluminium ou de zirconium, de colorants acides tels

que les colorants halogéno-acides, azoïques ou anthraquinoniques, les pigments enrobés par des composés siliconés tels que des polydiméthylsiloxanes et/ou par des polymères, notamment des polyéthylènes.

**32.** Composition selon la revendication 30 ou 31 telle que les pigments sont présents à une teneur allant de 0,05 à 80 %, de préférence allant de 0,5 à 50 %.en poids, par rapport au poids total de la composition.

**33.** Composition selon l'une quelconque des revendications 30 à 32 telle que les charges sont choisies parmi le talc, le mica, la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses synthétiques, les microéponges et les microbilles de résine de silicone, les oxydes de zinc et de titane, les oxydes de zirconium ou de cérium, le carbonate de calcium précipité, le carbonate et l'hydrocarbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone.

**34.** Composition selon l'une quelconque des revendications 30 à 33 telle que les charges sont présentes à une teneur allant de 0,1 à 99,9 %, de préférence allant de 70 à 99,9%, en poids, par rapport au poids total de la composition.

**35.** Composition selon l'une quelconque des revendications 30 à 34 telle que la phase pulvérulente comprend des nacres.

**36.** Composition selon la revendication précédente telle que les nacres sont présentes à une teneur allant de 0,05 à 80% en poids, de préférence allant de 2 à 50% en poids, par rapport au poids total de la composition.

**37.** Composition selon l'une quelconque des revendications 30 à 36 telle que la phase pulvérulente comprend des paillettes.

**38.** Composition selon l'une quelconque des revendications précédentes telle que la phase pulvérulente est présente à une teneur d'au moins 50% en poids, de préférence encore d'au moins 70% en poids, par rapport au poids total de la composition.

**39.** Composition selon l'une quelconque des revendications précédentes telle qu'elle se présente sous la forme de fards à paupières, de fards à joues, de poudres du visage et du corps, d'anticernes, de produits de maquillage du corps.

**40.** Procédé cosmétique de maquillage ou de soin des matières kératiniques des êtres humains, en particulier de la peau et du corps, comprenant l'application sur ces matières d'une composition telle que définie à l'une quelconque des revendications 1 à 39.

**41.** Utilisation d'au moins une composition à phase continue aqueuse, dans une composition de maquillage et/ou de soin sous forme de poudre, dans le but d'améliorer l'hydratation sur les matières kératiniques conférée par ladite composition.

**42.** Utilisation d'au moins une composition à phase continue aqueuse, dans une composition de maquillage et/ou de soin sous forme de poudre, dans le but d'améliorer le développement de la couleur au sein de ladite composition.

**43.** Procédé de préparation d'une composition telle que définie à l'une quelconque des revendications 1 à 39 comprenant les étapes suivantes :

a°) mélange des composés pulvérulents,
b°) broyage du mélange obtenu en a°) en particules fines,
c°) ajout du liant et mélange jusqu'à obtention d'une composition homogène.